# EUROPEAN PATENT SPECIFICATION

(11) **EP 3 767 596 B1**
(45) Date of publication and mention of the grant of the patent: **28.10.2026**
(21) Application number: 18914529.5
(22) Date of filing: 13.04.2018
(51) Int. Cl.: A61B 5/11, A61B 5/00, G01P 3/481

(54) **METHOD FOR CALCULATING CADENCE OF BICYCLE, WEARABLE DEVICE, AND STORAGE MEDIUM**
VERFAHREN ZUR BERECHNUNG DER TRITTFREQUENZ EINES FAHRRADS, TRAGBARE VORRICHTUNG UND SPEICHERMEDIUM
PROCÉDÉ DE CALCUL DE FRÉQUENCE DE PÉDALAGE DE VÉLO, DISPOSITIF POUVANT ÊTRE PORTÉ, ET SUPPORT D'INFORMATIONS

(43) Date of publication of application: 20.01.2021
(73) Proprietor: HUAWEI TECHNOLOGIES CO., LTD., Shenzhen, Guangdong 518129 (CN)
(72) Inventor: CHEN, Yixin, Shenzhen, Guangdong 518129 (CN); DONG, Chen, Shenzhen, Guangdong 518129 (CN); CHEN, Xiaohan, Shenzhen, Guangdong 518129 (CN); WANG, Yu, Shenzhen, Guangdong 518129 (CN)
(74) Representative: Gill Jennings & Every LLP
(86) International application number: PCT/CN2018/083031
(87) International publication number: WO 2019/196106

(56) References cited:
- WO-A1-2017/057037
- CN-A- 106 139 559
- CN-A- 106 663 343
- CN-A- 106 950 398
- CN-U- 205 409 997
- CN-U- 205 548 573
- US-A1- 2013 205 896
- US-A1- 2015 087 995
- US-A1- 2015 099 945
- YUTING ZHANG ET AL: "Monitoring walking and cycling of middle-aged to older community dwellers using wireless wearable accelerometers", ENGINEERING IN MEDICINE AND BIOLOGY SOCIETY (EMBC), 2013 34TH ANNUAL INTERNATIONAL CONFERENCE OF THE IEEE, IEEE, 28 August 2012 (2012-08-28), pages 158 - 161, XP032462884, ISSN: 1557-170X, DOI: 10.1109/EMBC.2012.6345895
- RALUCA MARIN-PERIANU ET AL: "A performance analysis of a wireless body-area network monitoring system for professional cycling", PERSONAL AND UBIQUITOUS COMPUTING, SPRINGER VERLAG, LO, vol. 17, no. 1, 27 November 2011 (2011-11-27), pages 197 - 209, XP035156504, ISSN: 1617-4917, DOI: 10.1007/S00779-011-0486-X
- XU JAMES Y ET AL: "Integrated Inertial Sensors and Mobile Computing for Real-Time Cycling Performance Guidance via Pedaling Profile Classification", IEEE JOURNAL OF BIOMEDICAL AND HEALTH INFORMATICS, IEEE, PISCATAWAY, NJ, USA, vol. 19, no. 2, 1 March 2015 (2015-03-01), pages 440 - 445, XP011574279, ISSN: 2168-2194, [retrieved on 20150303], DOI: 10.1109/JBHI.2014.2322871

## Description

### TECHNICAL FIELD

This application relates to the field of motion apparatuses, and in particular, to a method for calculating a tread frequency of a bicycle, a wearable device, and a storage medium.

### BACKGROUND

An accelerometer is usually mounted on a rotation carrier, for example, a bicycle wheel to obtain a rotational speed or a speed of the rotation carrier, for example, the bicycle wheel. A centripetal acceleration of the carrier during rotation is extracted based on an output signal of the accelerometer, and the rotational speed of the rotation carrier is obtained based on a relationship between a centripetal acceleration, a rotational speed, and a rotation radius. Alternatively, a periodic signal output by the accelerometer is detected in time domain and frequency domain. A rotation period or a rotation frequency of the carrier is obtained through calculation performed based on the periodic signal, to obtain the rotational speed or the speed of the rotation carrier. However, the accelerometer mounted on a bicycle needs to be independently mounted on the rotation carrier because the accelerometer is not a wearable device, and can transmit the output signal of the accelerometer only through communication, for example, Bluetooth. Therefore, the accelerometer is inconvenient for use.

A sensor, for example, an accelerometer is configured in a current wearable device. When a user wears the wearable device on a foot, the wearable device may calculate a current quantity of steps of the user based on a signal output by the sensor. However, the wearable device cannot be used to calculate a tread frequency or identify a riding state.

US 2015/099945 A1 describes determination of cycling cadence (crank revolutions per unit time). The cadence is detected from an acceleration signal of the rider's trunk (chest). Also described is a method distinguishing between different riding states - sitting and standing.

YUTING ZHANG ET AL: "Monitoring walking and cycling of middle-aged to older community dwellers using wireless wearable accelerometers", DOI: 10.1109/EMBC.2012.6345895, describes detecting walking and cycling events, and evaluating cycling parameters including cycling frequency. Detection of activity comprises a first stage of detection of dynamic or static activity and a second stage of detection of a particular type of dynamic activity such as walking or cycling. Dynamic activity is detected based on a large magnitude change in acceleration in a given time period, using a 3-axis acceleration signal. Cycling is distinguished from walking based on detecting a single peak in the frequency spectrum of the acceleration signal.

US 2013/205896 A1 describes a bicycle-mounted acceleration sensor, and local or remote analysis for determining cadence and travelling speed. Lateral acceleration oscillates with shifting weight as the rider pedals. The time between consecutive maxima or minima of the lateral acceleration can be measured as the inverse of the cadence. Additionally, the envelope of an acceleration signal varies according to a pedaling cycle. The cadence can alternatively be determined as the inverse of the time between consecutive maxima or minima in the envelope.

### SUMMARY

This application provides a method for calculating a tread frequency of a bicycle, a wearable device, and a storage medium, to resolve a problem in the prior art that a wearable device cannot calculate a tread frequency or automatically identify a riding state.

A first aspect of this application provides a computer-implemented method for calculating a tread frequency of a bicycle, as set out in claim 1.

In a solution provided in this application, when it is determined that the first state includes the riding state, the feature point set whose time distribution of the feature point meets the preset periodic distribution condition and whose amplitude meets the physical rule is determined from the acceleration signal. Then, when it is further determined that the feature point subset in the feature point set meets the preset reasonableness condition, it is determined that the first state is the riding state, and then the quantity of turns and the tread frequency that are in the first time window are calculated based on the feature point subset. By using this solution, after a user wears a wearable device on a leg, a quantity of turns and a tread frequency that are of a rotation carrier can be accurately calculated without a need to pre-obtain a parameter of the rotation carrier (for example, the bicycle) or mount the wearable device on the rotation carrier.

In some possible designs, the obtaining a first state of an acceleration signal in a first time window includes:
obtaining a three-axis acceleration signal;
calculating a combined acceleration of acceleration signals, of all axes, in the three-axis acceleration signal, where the combined acceleration is a square root of a sum of squares of a three-axis acceleration; and
determining the first state based on the combined acceleration, where the first state of the acceleration signal in the first time window may be a static state, a running state, a walking state, the riding state, or another state, and the first state is determined based on the combined acceleration, so that a state of the three-axis acceleration signal in the first time window can be preliminarily determined and screened.

In some possible designs, the feature point set includes a maximum value point set and a minimum value point set. Both time distribution of a maximum value point in the maximum value point set and time distribution of a minimum value point in the minimum value point set meet the preset periodic distribution condition, and the amplitude meets the related physical rule. This means that maximum value points and minimum value points are reasonable to be used to subsequently calculate the quantity of turns and the tread frequency.

In some possible designs, the determining a feature point set from the acceleration signal includes:
determining a first maximum value point set and a first minimum value point set from the acceleration signal;
calculating a time ratio and an amplitude ratio, of a first minimum value point and a second minimum value point, relative to a first maximum value point, where the first maximum value point is a maximum value point in the first maximum value point set, both the first minimum value point and the second minimum value are minimum value points in the first minimum value point set, and the first minimum value and the second minimum value are extreme value points adjacent to the first maximum value point; and
when the time ratio falls within a first value range and the amplitude ratio falls within a second value range, determining that time distribution of the first maximum value point meets the preset reasonableness condition, and obtaining the maximum value point set and the minimum value point set, where optionally, a preset threshold or an adaptive threshold may be further set to determine reasonableness of a maximum value point in the maximum value point set.

In some possible designs, the time ratio is [t(x) - t(y1)]/[t(y2) - t(x)], and the amplitude ratio is [acc_f(x) - acc_f(y1)]/[acc_f(y2) - acc_f(x)].

In the foregoing, t(x) is a time point corresponding to the first maximum value point x1, t(y1) is a time point corresponding to the first minimum value point y1, t(y2) is a time point corresponding to the second minimum value point y2, acc_f(x) represents an amplitude value, of the acceleration signal, at x, acc_f(y1) represents an amplitude value, of the acceleration signal, at y1, and acc_f(y2) represents an amplitude value, of the acceleration signal, at y2. If filtering processing is performed on the acceleration signal, when the time ratio and the amplitude ratio are calculated, acc_f(x), acc_f(y1), and acc_f(y2) are all amplitude values of an acceleration signal obtained after the filtering processing. If the filtering processing is not performed on the acceleration signal, when the time ratio and the amplitude ratio are calculated, acc_f(x), acc_f(y1), and acc_f(y2) are all amplitude values of the original acceleration signal. This is not specifically limited in this application.

In some possible designs, after the obtaining the maximum value point set and the minimum value point set, the method further includes:
selecting a group of maximum value points from the maximum value point set; and
determining reasonableness of the group of maximum value points based on the preset reasonableness condition, where the preset reasonableness condition includes at least one of the following items:
   a first ratio of an average value of the combined acceleration to a quantity of turns is greater than a first threshold; or
   a first standard deviation of a time interval between adjacent maximum value points is less than a second threshold; or
   a second ratio of a quantity of valid maximum value points to a quantity of original maximum value points is greater than a third threshold; or
   a second standard deviation of a difference between amplitude values of maximum value points is less than a fourth threshold; or
   a quantity of turns corresponding to a speed less than a first value is not greater than a first quantity of turns, a quantity of turns corresponding to a speed greater than the first value and less than a second value is not greater than a second quantity of turns, and the first quantity of turns is less than the second quantity of turns.

In some possible designs, the feature point set is an intersection point set, an intersection point in the intersection point set is an intersection point between the acceleration signal and an average value, and the average value is an average value of accelerations at all time points in the acceleration signal. Time distribution of these intersection points meets the preset periodic distribution condition, and amplitude meets the related physical rule.

In some possible designs, the acceleration signal is a gravity axis acceleration signal in the three-axis acceleration signal. There is almost no needed signal in an axis in a direction from the user's left to right. Therefore, the gravity axis acceleration signal in the three-axis acceleration signal may be used as the acceleration signal. It can be learned that only an acceleration signal in an axis in a gravity direction is used. This helps reduce impact caused by noise and reduce processing difficulty.

In some possible designs, smoothing processing is performed on the acceleration signal in a moving average filtering manner, and a high-frequency interference signal in the acceleration signal is removed in an infinite impulse response Butterworth low-pass filtering manner. A filtering manner includes but is not limited to the foregoing filtering manners, and another low-pass filtering manner may also be used.

A second aspect of this application provides a wearable device, and the wearable device has functions of implementing the method for calculating a tread frequency of a bicycle and implementing automatic detection of the riding state that are provided in the first aspect. The functions may be implemented by hardware, or may be implemented by hardware by executing corresponding software. The hardware or the software includes one or more modules corresponding to the foregoing functions, and the module may be software and/or hardware.

According to a third aspect, this application provides a computer-readable storage medium, and the computer-readable storage medium stores an instruction. When the instruction is run on a computer, the computer is enabled to perform the method in the first aspect.

According to a fourth aspect, this application provides a computer program product including an instruction, and when the computer program product runs on a computer, the computer is enabled to perform the method in the first aspect.

### BRIEF DESCRIPTION OF DRAWINGS

FIG. 1a is a schematic diagram of riding a bicycle by wearing a wearable device according to an embodiment of this application;
FIG. 1b is a schematic diagram of an original foot signal during walking collected by a wearable device according to an embodiment of this application;
FIG. 1c is a schematic diagram of an original foot signal during running collected by a wearable device according to an embodiment of this application;
FIG. 2 is a flowchart of a method for calculating a tread frequency of a bicycle according to an embodiment of this application;
FIG. 3a is a schematic diagram of a combined acceleration signal in a static state, a combined acceleration signal in a walking state, and a combined acceleration signal in a running state according to an embodiment of this application;
FIG. 3b is a schematic diagram of a combined acceleration signal in a riding state according to an embodiment of this application;
FIG. 3c is a schematic diagram of a combined acceleration signal in another state according to an embodiment of this application;
FIG. 4 is a schematic diagram of distribution of extreme value points of a main-axis acceleration signal according to an embodiment of this application;
FIG. 5 is a schematic diagram of an original three-axis acceleration signal according to an embodiment of this application;
FIG. 6 is a schematic diagram of comparison between an original three-axis acceleration signal and a filtered three-axis acceleration signals according to an embodiment of this application;
FIG. 7 is a schematic diagram of distribution of extreme value points of a main-axis signal according to an embodiment of this application;
FIG. 8a is a schematic diagram of a riding result of an outdoor bicycle according to an embodiment of this application;
FIG. 8b is a schematic diagram of a riding result of an outdoor bicycle according to an embodiment of this application;
FIG. 8c is a schematic diagram of a riding result of a stationary bicycle according to an embodiment of this application;
FIG. 8d is a schematic diagram of a riding result of a stationary bicycle according to an embodiment of this application;
FIG. 9 is a schematic structural diagram of a wearable device according to an embodiment of this application; and
FIG. 10 is a schematic structural diagram of a wearable device according to an embodiment of this application.

### DESCRIPTION OF EMBODIMENTS

In the specification, claims, and accompanying drawings of this application, the terms "first", "second", and the like are intended to distinguish between similar objects, but do not necessarily indicate a specific order or sequence. It should be understood that data used in such a way are interchangeable in proper circumstances, so that embodiments described herein can be implemented in other orders than the order illustrated or described herein. In addition, the terms "include", "have", or any other variant thereof are intended to cover non-exclusive inclusion. For example, a process, a method, a system, a product, or a device that includes a series of steps or modules is not necessarily limited to the steps or modules that are expressly listed, but may include another step or module not expressly listed or inherent to the process, the method, the product, or the device. The module division in this application is merely logical division, and there may be another division during implementation in actual application. For example, a plurality of modules may be combined or integrated into another system, or some features may be ignored or not performed. In addition, the displayed or discussed mutual couplings or direct couplings or communication connections may be implemented through some interfaces. The indirect couplings or communication connections between the modules may be implemented in electric or another form, and this is not limited in this application. In addition, modules or sub-modules described as separate components may be or may not be physically separated, or may be or may not be physical modules, or may be grouped into a plurality of circuit modules. Objectives of the solutions of the embodiments of this application may be achieved by selecting some or all of the modules according to an actual requirement.

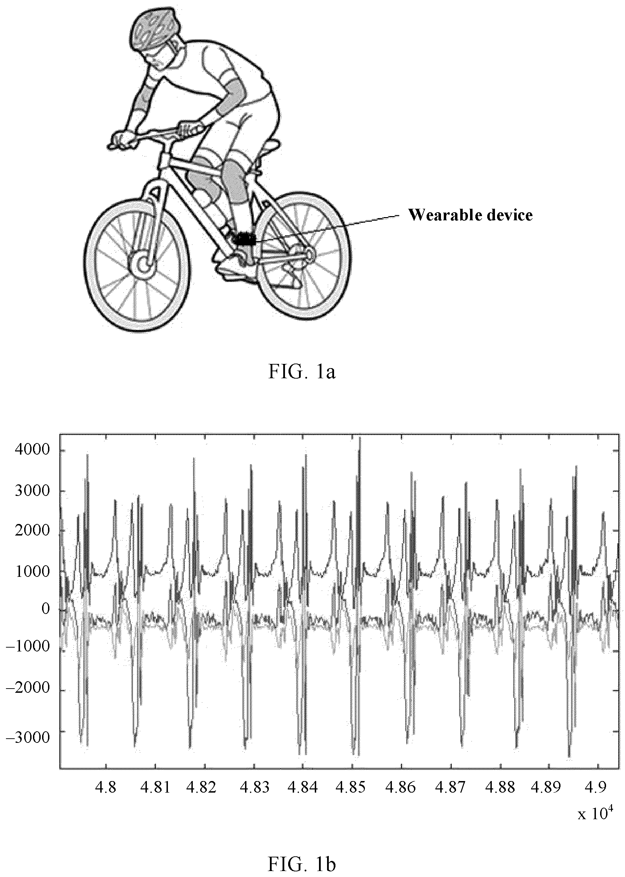

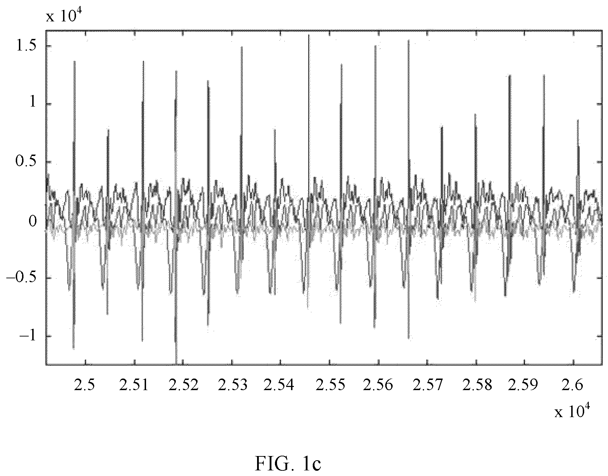

Embodiments of this application provide a method for calculating a tread frequency of a bicycle, a wearable device, and a storage medium that are applied to the field of motion apparatuses, for example, devices having rotation carriers such as the bicycle and an indoor stationary bicycle. This can resolve a problem in the prior art that a tread frequency cannot be accurately calculated through a wearable fabric in an existing mechanism. Detailed descriptions are provided below. FIG. 1a is a schematic diagram in which a user wearing a wearable device rides a bicycle. FIG. 1b shows an original foot signal during walking, of the user, collected by the wearable device in FIG. 1a. FIG. 1c shows an original foot signal during running, of the user, collected by the wearable device in FIG. 1a. In this application, a quantity of turns and a tread frequency are mainly calculated based on an original foot signal during running, for example, based on the original foot signal during running shown in FIG. 1c.

The wearable device in the embodiments of this application may be smart wearables, a sports foot band, a smart foot band, clothes, shoes, or the like.

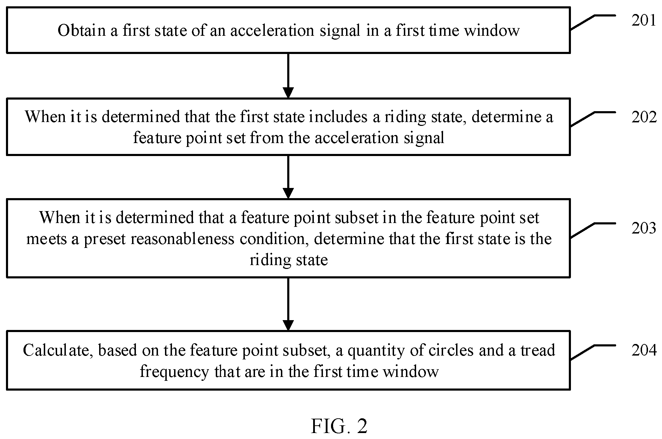

Referring to FIG. 2, the following describes an example of a method for calculating a tread frequency of a bicycle according to this application. The method includes the following steps.

201: Obtain a first state of an acceleration signal in a first time window.

The acceleration signal in this embodiment of this application may be sampled at a fixed sampling frequency. For example, if a length of a time window is 5s, and the sampling frequency is 100 Hz, a signal in each axis has 500 time points and corresponding amplitude values (namely, acceleration values).

In some implementations, obtaining the first state of the acceleration signal in the first time window includes the following steps:
(a) Obtain a three-axis acceleration signal.
(b) Calculate a combined acceleration of acceleration signals, of all axes, in the three-axis acceleration signal.
   The combined acceleration may be a square root of a sum of squares of a three-axis acceleration. In other words, the combined acceleration is sqrt(x^2 + y^2 + z^2), and sqrt represents calculating the square root.
(c) Determine the first state based on the combined acceleration.

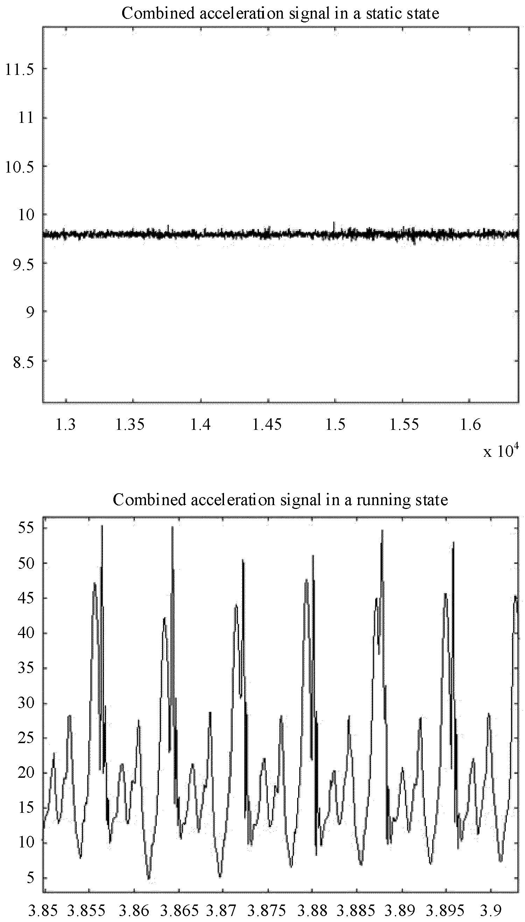

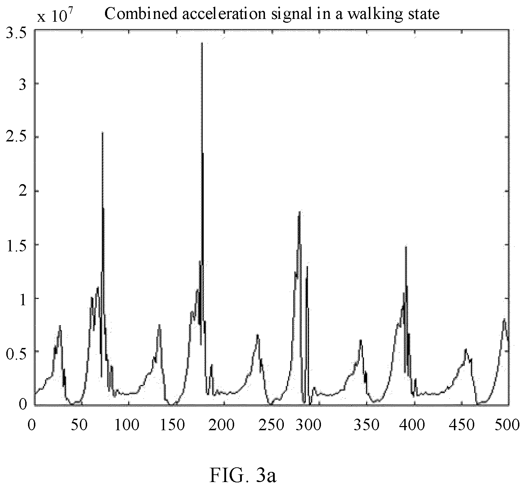

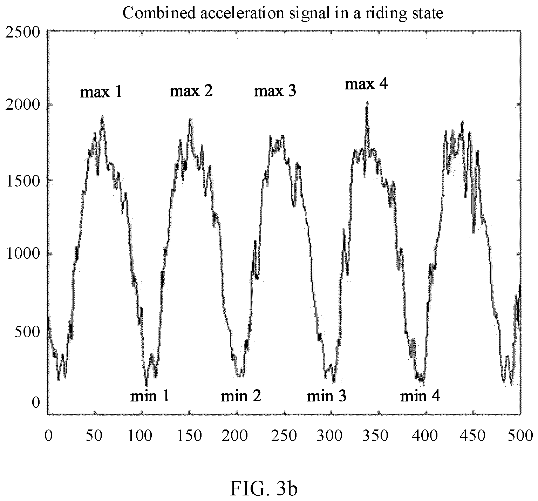

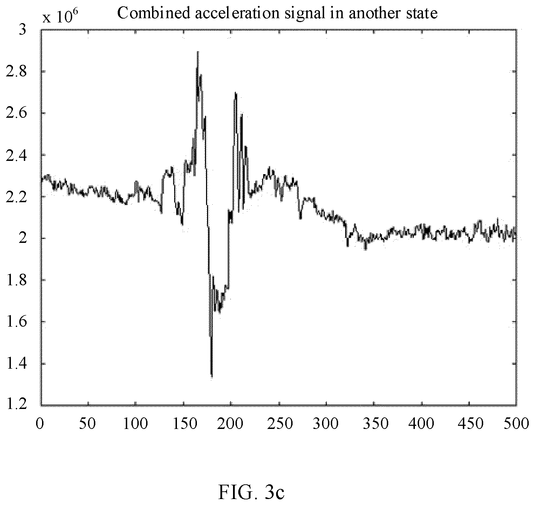

For example, the first state of the acceleration signal in the first time window may be a static state, a running state, and a walking state shown in FIG. 3a, or may be a riding state shown in FIG. 3b, or may be another state shown in FIG. 3c. For example, for the riding state shown in FIG. 3b, extreme value points max 1, max 2, max 3, max 4, min 1, min 2, min 3, and min 4 are characterized by periodic time distribution. The first state is determined based on the combined acceleration, so that a state of the three-axis acceleration signal in the first time window can be preliminarily determined and screened.

In some implementations, it is considered that the three-axis acceleration signal includes a signal that is really needed for calculating the tread frequency and a signal that is not needed. In this embodiment of this application, to accurately calculate the tread frequency and reduce calculation complexity of the tread frequency, a main-axis signal may be selected from the three-axis acceleration signal as the acceleration signal used to calculate the tread frequency. Oscillation is generated during riding of a user wearing a wearable device, and irregular noise caused by the oscillation exists in a three-axis direction. However, there is almost no needed signal of an axis in a direction from the user's left to right. Therefore, a gravity axis acceleration signal in the three-axis acceleration signal may be used as the acceleration signal. It can be learned that only an acceleration signal of an axis in a gravity direction is used. This helps reduce impact caused by the noise and reduce processing difficulty. For example, if a length of the first time window is 5s, and the sampling frequency is 100 Hz, the signal of each axis has 500 time points and the corresponding amplitude values (namely, the acceleration values). The acceleration values of the 500 time points are averaged to obtain an average value and a standard deviation. An acceleration signal of each axis in the three-axis acceleration signal has one average value and one standard deviation in the first time window. An acceleration signal of one axis may be selected as the main-axis signal based on three average values and three standard deviations.

Then, whether a main-axis acceleration signal is smooth is determined based on a quantity of extreme value points of the combined acceleration. If the main-axis acceleration signal is not smooth, smoothing processing is performed on the main-axis acceleration signal in a moving average filtering manner. Whether there is a high-frequency interference signal in the main-axis acceleration signal is determined. If there is the high-frequency interference signal, the high-frequency interference signal is removed from the acceleration signal in an infinite impulse response (infinite impulse response digital filter, IIR) Butterworth low-pass filtering manner.

202: When it is determined that the first state includes the riding state, determine a feature point set from the acceleration signal.

Time distribution of a feature point in the feature point set meets a preset periodic distribution condition, and an amplitude meets a related physical rule. For example, for the riding state shown in FIG. 3b, the feature points max 1, max 2, max 3, max 4, min 1, min 2, min 3, and min 4 are characterized by periodic time distribution, and amplitude meets the related physical rule.

In some implementations, the feature point set may be an intersection point set, an intersection point in the intersection point set is an intersection point between the acceleration signal and an average value, and the average value is an average value of accelerations at all time points in the acceleration signal. Time distribution of these intersection points meets the preset periodic distribution condition, and amplitude meets the related physical rule.

Alternatively, in some implementations, the feature point set includes a maximum value point set and a minimum value point set, both time distribution of a maximum value point in the maximum value point set and time distribution of a minimum value point in the minimum value point set meet the preset periodic distribution condition, and the amplitude meets the related physical rule.

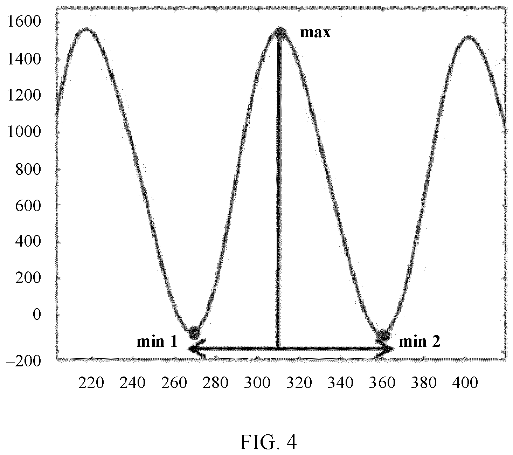

The extreme value point (including the maximum value point and the minimum value point) is a point whose first-order derivative value is 0, for example, a main-axis acceleration signal shown in FIG. 4. The main-axis acceleration signal includes a maximum value point max and minimum value points min 1 and min 2.

When the maximum value point and the minimum value point are searched in the main-axis acceleration signal, a time interval between two adjacent maximum value points needs to meet a preset time condition, and a maximum value point that meets the time condition is preliminarily obtained through screening. Then, the minimum value point min is found between the two maximum value points. In this way, two time phases of a period are preliminarily found. For the time condition, the time interval between the adjacent maximum value points is usually relatively long. Considering that a time period for a person to ride around is not very short, it may be considered that the time interval is greater than 0.2s. If the time interval between the two adjacent maximum value points does not meet the time condition, a maximum value point with a larger amplitude value is selected as a candidate maximum value point, and a maximum value point with a smaller amplitude value is discarded. For example, the main-axis acceleration signal shown in FIG. 4 includes the maximum value point max and the minimum value points min 1 and min 2, and one period includes min 1→max 1 and max 1→min 2.

In some implementations, the step of determining the feature point set from the acceleration signal may include step (a) to step (c).
(a) Determine a first maximum value point set and a first minimum value point set from the acceleration signal.
   Maximum value points included in the first maximum value point set are all original maximum value points, and minimum value points included in the first minimum value point set are original minimum value points.
(b) Calculate a time ratio and an amplitude ratio, of a first minimum value point and a second minimum value point, relative to a first maximum value point.

The first maximum value point is a maximum value point in the first maximum value point set, both the first minimum value point and the second minimum value are minimum value points in the first minimum value point set, and the first minimum value and the second minimum value are extreme value points adjacent to the first maximum value point.

In some implementations, a calculation formula of the time ratio is [t(x) - t(y1)]/[t(y2) - t(x)], and a calculation formula of the amplitude ratio is [acc_f(x) - acc_f(y1)]/[acc_f(y2) - acc_f(x)].

In the foregoing, t(x) is a time point corresponding to the first maximum value point x1, t(y1) is a time point corresponding to the first minimum value point y1, t(y2) is a time point corresponding to the second minimum value point y2, acc_f(x) represents an amplitude value, of the acceleration signal, at x, acc_f(y1) represents an amplitude value, of the acceleration signal, at y1, and acc_f(y2) represents an amplitude value, of the acceleration signal, at y2. It should be noted that, if filtering processing is performed on the acceleration signal, when the time ratio and the amplitude ratio are calculated, acc_f(x), acc_f(y1), and acc_f(y2) are all amplitude values of an acceleration signal obtained after the filtering processing. If the filtering processing is not performed on the acceleration signal, when the time ratio and the amplitude ratio are calculated, acc_f(x), acc_f(y1), and acc_f(y2) are all amplitude values of the original acceleration signal. This is not specifically limited in this application.
(c) When the time ratio falls within a first value range and the amplitude ratio falls within a second value range, determine that time distribution of the first maximum value point meets a preset reasonableness condition, which indicates that the first maximum value point is a valid maximum value point. Through step (a) to step (c) in this implementation, reasonableness of maximum value points in the maximum value point set may be determined in sequence, and finally the maximum value point set and the minimum value point set may be obtained.

In some implementations, a preset threshold or an adaptive threshold may be further set to determine reasonableness of the maximum value point in the maximum value point set.

203: When it is determined that a feature point subset in the feature point set meets the preset reasonableness condition, determine that the first state is the riding state.

After the maximum value point set is obtained by determining reasonableness of a single maximum value point, reasonableness of a group of valid maximum value points in the maximum value point set further needs to be determined, to further determine whether the first state in the first time window is the riding state. For determining reasonableness of a group of maximum value points, in some implementations, a condition for determining the reasonableness of the group of valid maximum value points based on a periodic feature of a waveform of the acceleration signal (namely, a principle that parameters change slightly in a period within a short time period) may include the following step (a) to step (e).
(a) Determine based on a first ratio of an average value of the combined acceleration to a quantity of turns. For example, the calculated first ratio (usually, a larger quantity of turns indicates a larger average value) is greater than a first threshold.
(b) On a premise that the time distribution of the maximum value point is even, determine based on a first standard deviation of the time interval between the adjacent maximum value points. The first standard deviation is less than a specified second threshold.
(c) Determine based on a second ratio of a quantity of valid maximum value points to a quantity of original maximum value points. Because there are few invalid maximum value points in the maximum value point set, the second ratio may be set to be greater than a third threshold. Optionally, a value range of the third threshold may be from 0.2 to 0.6. This is not specifically limited in this embodiment of this application.
(d) Determine based on a second standard deviation (an amplitude change is relatively small) of a difference between amplitude values of maximum value points. The second standard deviation is less than a fourth threshold.
(e) Determine based on a relationship between the quantity of turns and a speed range (the relationship is consistent with an initially calculated speed range). For that a quantity of turns corresponding to a speed less than a first value is not greater than a first quantity of turns, for example, it may be set that the first quantity of turns at a low speed is less than or equal to 4. For a quantity of turns corresponding to a speed greater than the first value and less than a second value is not greater than a second quantity of turns, for example, it may be set that the second quantity of turns at a non-high speed is less than or equal to 8.5. The quantity of turns is a quantity of reasonable periods that are found, and may also be referred to as a quantity of reasonable maximum value points. A specific name is not limited in this application.

It can be learned that, after step (a) to step (e) are performed to determine the reasonableness of the group of valid maximum value points, a range of the valid maximum value points can be further narrowed and be more accurate. This improves data accuracy and further determines whether the first state of the acceleration signal in the first time window is a reasonable riding state.

After it is determined that the first state of the acceleration signal is the riding state, a state in a previous time window further needs to be corrected based on a state of the acceleration signal in the previous time window. A second state of the acceleration signal in a second time window and a third state of the acceleration signal in a third time window are obtained. The second time window, the third time window, and the first time window are adjacent and arranged in ascending order in time domain.

Then, the third state is corrected based on the second state and the first state. For example, when both the second state and the first state are non-riding states, and the third state is the riding state, the state of the acceleration signal in the third time window is corrected to the non-riding state, and a quantity of turns in the third time window is set to 0.

For another example, when both the second state and the first state are riding states, and the third state is the non-riding state, the state of the acceleration signal in the third time window is corrected to the riding state. The second quantity of turns is calculated based on a feature point set of the acceleration signal in the third time window. When a ratio of the second quantity of turns to the first quantity of turns falls within a third value range, it is determined that the state of the acceleration signal in the third time window is the riding state. Because an originally recorded third state is the non-riding state, an originally recorded quantity of turns in the third time window is 0. After the second quantity of turn in the third time window is recalculated this time, a recorded quantity of turns in the third time window may be updated to the second quantity of turn. For example, the quantity of turns may be calculated based on a valid maximum value point in the third time window, and then the quantity of turns in the third time window is compared with a quantity of turns in the first time window. If a ratio is within a specific value range, for example, the ratio is between 0.8 and 1.2, it may be considered that the state in the third time window is the riding state, and the originally recorded quantity 0 of turns in the third time window is replaced with the currently calculated quantity of turns in the third time window.

It can be learned that, after the state in the second time window (namely, the previous time window) is corrected, there may not be a case in which there are two non-riding states and one riding state between the two non-riding states, or a case in which there is another non-static state during riding. It should be noted that states and turns in three consecutive time windows need to be used herein. Therefore, the state in the third time window (namely, a previous time window of the first time window) is corrected in the first time window.

204: Calculate, based on the feature point subset, the quantity of turns and a tread frequency that are in the first time window.

In some implementations, quantity of turns = motion time period x sampling frequency/single-turn period. The motion time period may be in a unit of a time window.

The single-cycle period may be obtained based on a time interval between adjacent feature points. For example, the single-cycle period may be obtained by taking a median from a time interval between adjacent valid maximum value points.

A calculation formula of the tread frequency is: tread frequency = 60 x sampling frequency/single-turn period x motion time period ratio. The motion time period ratio is a ratio of a time window occupied by an actual riding state to a total motion time period.

In the solution provided in this application, the riding state can be automatically detected. When it is determined that the first state includes the riding state, the feature point set whose time distribution of the feature point meets the preset periodic distribution condition and whose amplitude meets the related physical rule is determined from the acceleration signal. Then, when it is further determined that the feature point subset in the feature point set meets the preset reasonableness condition, it is determined that the first state is the riding state, and then the quantity of turns and the tread frequency that are in the first time window are calculated based on the feature point subset. By using this solution, after the user wears the wearable device on a leg, a quantity of turns and a tread frequency that are of a rotation carrier can be accurately calculated without a need to pre-obtain a parameter of the rotation carrier (for example, the bicycle) or mount the wearable device on the rotation carrier.

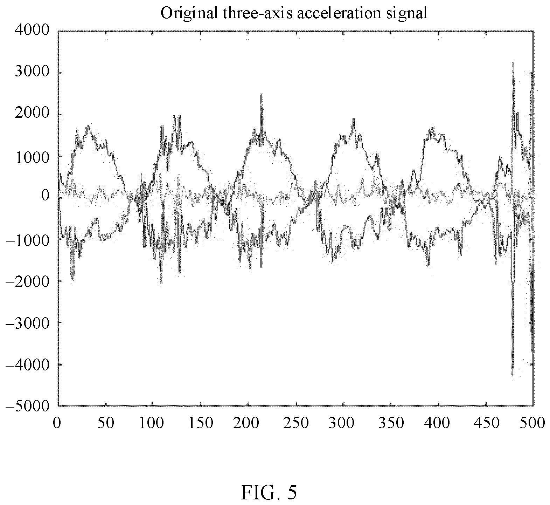

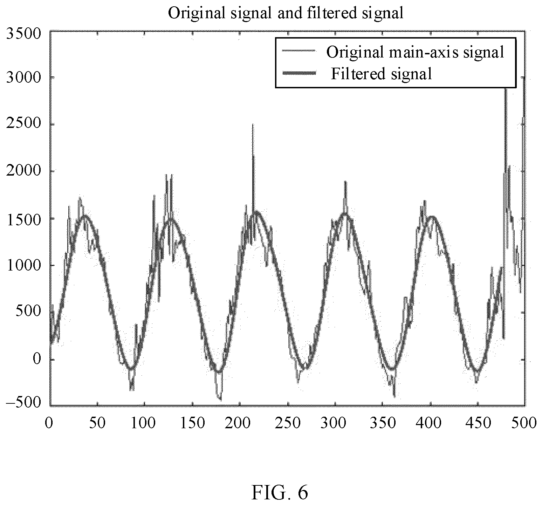

For ease of understanding, the following uses a stationary bicycle as an example to describe a method for calculating a tread frequency during riding in the embodiments of this application. As shown in FIG. 5, FIG. 5 is a segment of 5-second original acceleration signal input into a wearable device. An acceleration signal, of an axis, with a maximum amplitude average value and a maximum standard deviation is selected from the original acceleration signal shown in FIG. 5 as a main-axis acceleration signal (which may be referred to as a main-axis signal for short). Because noise in the main-axis signal is relatively large, to facilitate subsequent data analysis, in this embodiment of this application, an IIR may be used to perform filtering processing on the main-axis signal. For a main-axis signal obtained after the filtering processing, refer to a schematic diagram of a signal shown in FIG. 6.

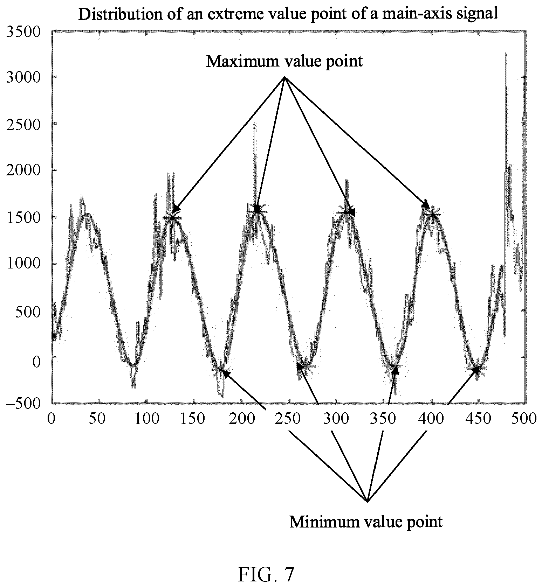

Based on the main-axis signal shown in FIG. 6, a maximum value point and a minimum value point are extracted from the main-axis signal. Finally, four maximum value points and four minimum value points are extracted in total. An extraction result is shown in FIG. 7. As shown in FIG. 7, the four maximum value points whose amplitude values are about 1500 are respectively [127, 217, 310, 401], and the four minimum value points whose amplitude values are about 0 are respectively [178, 268, 359, 449]. The four maximum value points shown in FIG. 7 are all valid maximum value points. For a manner of determining the maximum value point and the minimum value point, refer to the description of step 202 in which the reasonableness of the single maximum value point is determined according to that the time distribution meets the preset reasonableness condition, and refer to the condition, described in step 203, used to determine the reasonableness of the group of valid maximum value points according to step (a) to step (e). A specific determining process is not described herein again. The minimum value point is determined based on the valid maximum value point.

After the four maximum value points and the four minimum value points shown in FIG. 7 are extracted, the following calculates a single-cycle period, a quantity of cycles, and a tread frequency based on a time interval between the maximum value points.
(1) Calculate the single-cycle period. Time intervals between the four maximum value points in FIG. 7 are sequentially [90, 93, 91]. A median 91 is taken from the three time intervals. In other words, the single-cycle period is 91.
(2) Calculate the quantity of turns, where quantity of turns = motion time period x sampling frequency/single-cycle period. For example, if the motion time period is 5s, the single-cycle period is 91 points, and the sampling frequency is 100 Hz, the quantity of turns is 5 x 100/91 = 5.49.
(3) Calculate the tread frequency, where tread frequency = 60 x sampling frequency/single-cycle period x motion time period ratio = 60 x 100/91 x 1 = 65.93.

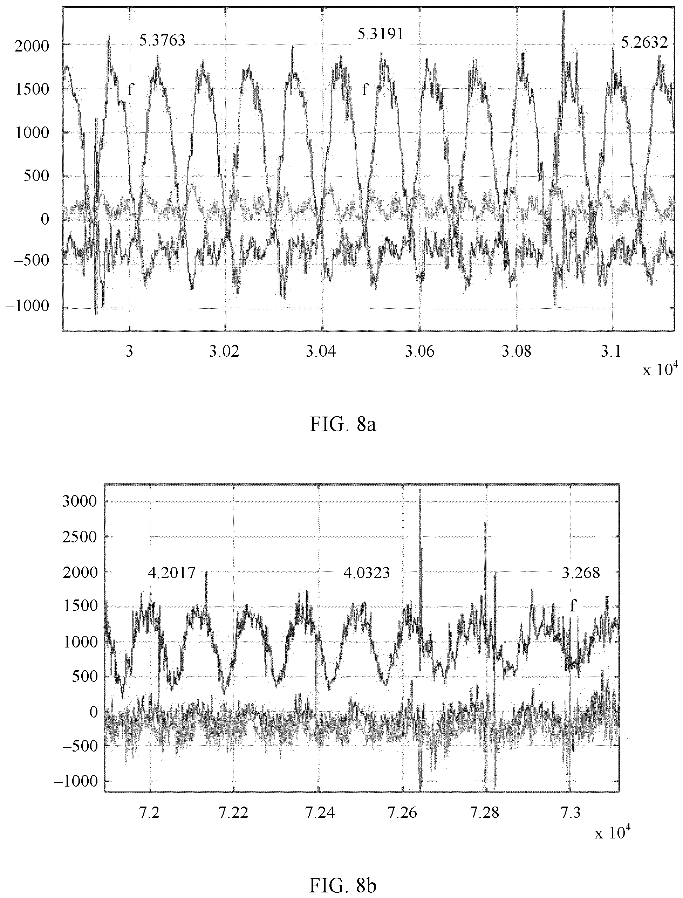

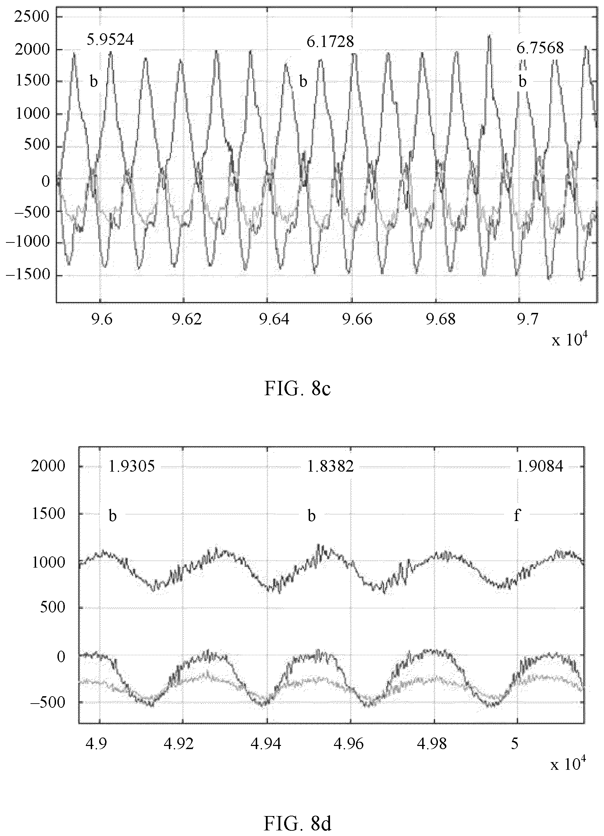

Finally, the tread frequency in this time window is 65.93 and the quantity of turns is 5.49. An integer result of the quantity of turns may be displayed. This is not specifically limited in this application. Finally, the quantity of cycles is used for statistics about a riding result of this test. For example, a riding result 1 of an outdoor bicycle is shown in FIG. 8a. According to the solution described in this embodiment of this application, a riding result 2 of the outdoor bicycle, a riding result 1 of a stationary bicycle, and a riding result 2 of the stationary bicycle shown in FIG. 8b to FIG. 8d may be further obtained.

It can be learned that in this embodiment of this application, the quantity of cycles and the tread frequency are accurately calculated by identifying a riding state. If a total quantity of turns is used for the statistics, a final data accuracy can reach 97.66%.

In some implementations, a calculation formula of the accuracy may be accuracy = 1 - abs (method statistics in the present invention - real quantity of turns)/real quantity of turns. In the foregoing, abs indicates that an absolute value is taken. According to a preliminary experiment, when an experiment condition is that 20 people ride the bicycle and the stationary bicycle, the accuracy on a bicycle side is about 92%, and the accuracy on a stationary bicycle side is 94%. A proportion of a case in which a non-riding state is incorrectly determined as the riding state to a total data duration is less than 0.5%.

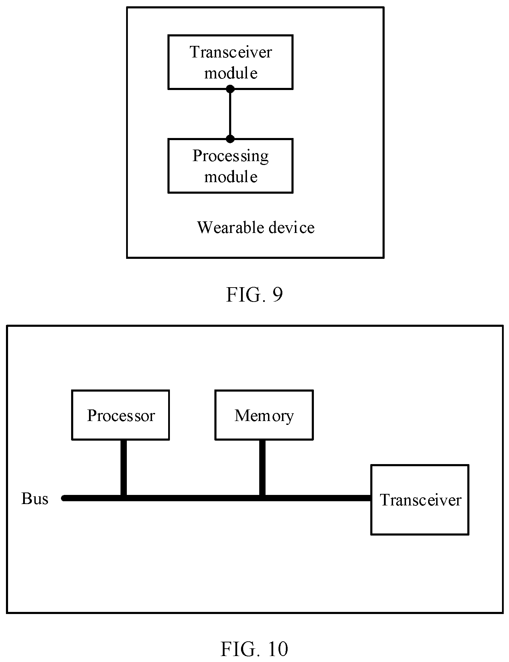

The foregoing describes the method for calculating a tread frequency of a bicycle in this application. The following describes a wearable device that performs the method for calculating a tread frequency of a bicycle. Referring to FIG. 9, the wearable device is described. The wearable device includes:
an obtaining module, configured to obtain a first state of an acceleration signal in a first time window; and
a processing module, configured to: when it is determined that the first state obtained by the obtaining module includes a riding state, determine a feature point set from the acceleration signal, where time distribution of a feature point in the feature point set meets a preset periodic distribution condition; when it is determined that a feature point subset in the feature point set meets a preset reasonableness condition, determine that the first state is the riding state; and calculate, based on the feature point subset, a quantity of turns and a tread frequency that are in the first time window.

In this embodiment of this application, when it is determined that the first state includes the riding state, the processing module determines, from the acceleration signal, the feature point set whose time distribution of the feature point meets the preset periodic distribution condition and whose amplitude meets a related physical rule. Then, when it is further determined that the feature point subset in the feature point set meets the preset reasonableness condition, it is determined that the first state is the riding state, and then the quantity of turns and the tread frequency that are in the first time window are calculated based on the feature point subset. By using this solution, after a user wears the wearable device on a leg, a quantity of turns and a tread frequency that are of a rotation carrier can be accurately calculated without a need to pre-obtain a parameter of the rotation carrier (for example, the bicycle) or mount the wearable device on the rotation carrier.

Optionally, in some embodiments of this application, the obtaining module is configured to:
obtain a three-axis acceleration signal;
calculate a combined acceleration of acceleration signals, of all axes, in the three-axis acceleration signal; and
determine the first state based on the combined acceleration.

Optionally, in some embodiments of this application, the feature point set includes a maximum value point set and a minimum value point set, both time distribution of a maximum value point in the maximum value point set and time distribution of a minimum value point in the minimum value point set meet the preset periodic distribution condition, and the amplitude meets the related physical rule.

Optionally, in some embodiments of this application, the processing module is configured to:
determine a first maximum value point set and a first minimum value point set from the acceleration signal;
calculate a time ratio and an amplitude ratio, of a first minimum value point and a second minimum value point, relative to a first maximum value point, where the first maximum value point is a maximum value point in the first maximum value point set, both the first minimum value point and the second minimum value are minimum value points in the first minimum value point set, and the first minimum value and the second minimum value are extreme value points adjacent to the first maximum value point; and
when the time ratio falls within a first value range and the amplitude ratio falls within a second value range, determine that time distribution of the first maximum value point meets the preset reasonableness condition, and obtain the maximum value point set and the minimum value point set.

In some implementations, the time ratio is [t(x) - t(y1)]/[t(y2) - t(x)], and the amplitude ratio is [acc_f(x) - acc_f(y1)]/[acc_f(y2) - acc_f(x)], where
t(x) is a time point corresponding to the first maximum value point x1, t(y1) is a time point corresponding to the first minimum value point y1, t(y2) is a time point corresponding to the second minimum value point y2, acc_f(x) represents an amplitude value, of the acceleration signal, at x, acc_f(y1) represents an amplitude value, of the acceleration signal, at y1, and acc_f(y2) represents an amplitude value, of the acceleration signal, at y2.

In some implementations, after obtaining the maximum value point set and the minimum value point set, the processing module is further configured to:
select a group of maximum value points from the maximum value point set; and
determine reasonableness of the group of maximum value points based on the preset reasonableness condition, where the preset reasonableness condition includes at least one of the following items:
   a first ratio of an average value of the combined acceleration to a quantity of turns is greater than a first threshold; or
   a first standard deviation of a time interval between adjacent maximum value points is less than a second threshold; or
   a second ratio of a quantity of valid maximum value points to a quantity of original maximum value points is greater than a third threshold; or
   a second standard deviation of a difference between amplitude values of maximum value points is less than a fourth threshold; or
   a quantity of turns corresponding to a speed less than a first value is not greater than a first quantity of turns, a quantity of turns corresponding to a speed greater than the first value and less than a second value is not greater than a second quantity of turns, and the first quantity of turns is less than the second quantity of turns.

After determining that the first state of the acceleration signal is the riding state, the processing module is further configured to:
obtain, through the obtaining module, a second state of the acceleration signal in a second time window and a third state of the acceleration signal in a third time window, where the second time window, the third time window, and the first time window are adjacent and arranged in ascending order in time domain; and
correct the third state based on the second state and the first state.

Optionally, in some embodiments of this application, the processing module is configured to:
when both the second state and the first state are non-riding states, and the third state is the riding state, correct the state of the acceleration signal in the third time window to the non-riding state, and set a quantity of turns in the third time window to 0; or
when both the second state and the first state are riding states, and the third state is a non-riding state, correct the state of the acceleration signal in the third time window to the riding state.

Optionally, in some embodiments of this application, when both the second state and the first state are the riding states, and the third state is the non-riding state, the processing module is further configured to:
calculate the second quantity of turns based on a feature point set of the acceleration signal in the third time window; and
when a ratio of the second quantity of turns to the first quantity of turns falls within a third value range, determine that the state of the acceleration signal in the third time window is the riding state, and update a recorded quantity of turns in the third time window to the second quantity of turns.

FIG. 10 is another schematic structural diagram of a wearable device according to an embodiment of this application. The wearable device may include at least one processor, at least one network interface or another communications interface, a memory, at least one communications bus, and at least one transceiver that are configured to implement connection and communication between these apparatuses. The processor is configured to execute an executable module stored in the memory, for example, a computer program. The memory may include a high speed random access memory (English full name: Random Access Memory, RAM for short), or may further include a non-volatile memory (non-volatile memory), for example, at least one magnetic disk memory. A communication connection between a system gateway and at least one another network element is implemented through the at least one network interface (which may be a wired interface or a wireless interface), and the internet, a wide area network, a local area network, a metropolitan area network, or the like may be used.

As shown in FIG. 10, in some implementations, the memory stores a program instruction, and the program instruction may be executed by the processor. The program instruction stored in the memory is invoked, so that the processor specifically executes program code that needs to be invoked when the service management method in the embodiments of this application is performed.

It should be noted that in the embodiment corresponding to FIG. 9 of this application, a physical device corresponding to the transceiver module may be the transceiver, and a physical device corresponding to the processing module may be the processor. Each apparatus shown in FIG. 9 may have a structure shown in FIG. 10. When one of the apparatuses has the structure shown in FIG. 10, the processor and the transceiver in FIG. 10 implement functions same as or similar to those of the processing module and the transceiver module that are provided in the foregoing apparatus embodiment corresponding to the apparatus, and the memory in FIG. 10 stores program code that needs to be invoked when the processor performs the method for calculating a tread frequency of a bicycle.

This application further provides a computer storage medium. The medium stores a program. When the program is executed, some or all of the steps in the method, for calculating a tread frequency of a bicycle, performed by the wearable device are included.

In the foregoing embodiments, a description of each embodiment has respective focuses. For a part that is not described in detail in an embodiment, refer to related descriptions in another embodiment.

A person skilled in the art may be clearly understood that, for convenient and brief description, for a detailed working process of the foregoing system, apparatus, and unit, refer to a corresponding process in the foregoing method embodiment. Details are not described herein again.

In the several embodiments provided in this application, it should be understood that the disclosed system, apparatus, and method may be implemented in another manner. For example, the described apparatus embodiment is merely an example. For example, division into the modules or units is merely logical function division and may be other division in an actual implementation. For example, a plurality of modules or components may be combined or integrated into another system, or some features may be ignored or not performed. In addition, the displayed or discussed mutual coupling or a direct coupling or a communication connection may be implemented by using some interfaces. An indirect coupling or a communication connection between the apparatuses or units may be implemented in an electronic form, a mechanical form, or in another form.

The modules described as separate parts may or may not be physically separate, and parts displayed as modules may or may not be physical modules, may be located in one position, or may be distributed on a plurality of network modules. Some or all of the modules may be selected based on an actual requirement to achieve the objectives of the solutions in the embodiments.

In addition, functional modules in the embodiments of this application may be integrated into one processing module, or each of the modules may exist alone physically, or two or more modules are integrated into one module. The integrated module may be implemented in a form of hardware, or may be implemented in a form of a software function module. When the integrated module is implemented in the form of a software function module and sold or used as an independent product, the integrated module may be stored in a computer-readable storage medium.

All or some of the foregoing embodiments may be implemented through software, hardware, firmware, or any combination thereof. When software is used to implement the embodiments, all or some of the embodiments may be implemented in a form of a computer program product.

The computer program product includes one or more computer instructions. When the computer program instructions are loaded and executed on a computer, the procedures or the functions according to the embodiments of this application are all or partially generated. The computer may be a general-purpose computer, a special-purpose computer, a computer network, or another programmable apparatus. The computer instructions may be stored in the computer-readable storage medium or may be transmitted from one computer-readable storage medium to another computer-readable storage medium. For example, the computer instructions may be transmitted from one website, computer, server, or data center to another website, computer, server, or data center wiredly (for example, a coaxial cable, an optical fiber, or a digital subscriber line (DSL)) or wirelessly (for example, infrared, radio, or microwave). The computer-readable storage medium may be any usable medium accessible by a computer, or a data storage device, for example, a server or a data center, integrating one or more usable media. The usable medium may be a magnetic medium (for example, a floppy disk, a hard disk, or a magnetic tape), an optical medium (for example, a DVD), a semiconductor medium (for example, a solid-state drive Solid State Disk (SSD)), or the like.

The technical solutions provided in this application are described in detail above. The principle and implementation of this application are described herein through specific examples. The description about the embodiments is merely provided to help understand the method and core ideas of this application.

## Claims

1. A computer-implemented method for calculating a tread frequency of a bicycle, wherein the method comprises:
determining (201) a first state of an acceleration signal in a first time window, wherein the step of determining (201) comprises:
obtaining a three-axis acceleration signal from a wearable device worn on a user's leg;
calculating a combined acceleration of acceleration signals, of all axes, in the three-axis acceleration signal; and
determining the first state based on the combined acceleration;
when it is determined that the first state is a non-riding state, recording a quantity of turns in the first time window as 0;
when it is preliminarily determined that the first state comprises a riding state, determining (202) a feature point set from the acceleration signal, wherein time distribution of a feature point in the feature point set meets a preset periodic distribution condition, and amplitude meets a physical rule, and when it is determined that a feature point subset in the feature point set meets a preset reasonableness condition, determining (203) that the first state is the riding state; and
calculating (204), based on the feature point subset, a quantity of turns and a tread frequency that are in the first time window,
wherein after the determining (203) that the first state of the acceleration signal is the riding state, the method further comprises:
determining a second state of the acceleration signal in a second time window and a third state of the acceleration signal in a third time window,
wherein the second time window, the third time window, and the first time window are adjacent and arranged in ascending order in time domain; and
when both the second state and the first state are riding states, and the third state is a non-riding state, correcting the state of the acceleration signal in the third time window by:
calculating a second quantity of turns based on a feature point set of the acceleration signal in the third time window; and
when a ratio of the second quantity of turns to the first quantity of turns falls within a third value range, correcting the state of the acceleration signal in the third time window to the riding state, and updating a recorded quantity of turns in the third time window to the second quantity of turns.

2. The method according to claim 1, wherein the feature point set comprises a maximum value point set and a minimum value point set, both time distribution of a maximum value point in the maximum value point set and time distribution of a minimum value point in the minimum value point set meet the preset periodic distribution condition, and the amplitude meets the physical rule.

3. The method according to claim 2, wherein the determining (202) a feature point set from the acceleration signal comprises:
determining a first maximum value point set and a first minimum value point set from the acceleration signal;
calculating a time ratio and an amplitude ratio, of a first minimum value point and a second minimum value point, relative to a first maximum value point, wherein the first maximum value point is a maximum value point in the first maximum value point set, both the first minimum value point and the second minimum value point are minimum value points in the first minimum value point set, and the first minimum value point and the second minimum value point are extreme value points adjacent to the first maximum value point; and
when the time ratio falls within a first value range and the amplitude ratio falls within a second value range, determining that time distribution of the first maximum value point meets the preset reasonableness condition, and obtaining the maximum value point set and the minimum value point set.

4. The method according to claim 3, wherein the time ratio is [t(x) - t(y1)]/[t(y2) - t(x)], and the amplitude ratio is [acc_f(x) - acc_f(y1)]/[acc_f(y2) - acc_f(x)], wherein
t(x) is a time point corresponding to the first maximum value point x1, t(y1) is a time point corresponding to the first minimum value point y1, t(y2) is a time point corresponding to the second minimum value point y2, acc_f(x) represents an amplitude value, of the acceleration signal, at x, acc_f(y1) represents an amplitude value, of the acceleration signal, at y1, and acc_f(y2) represents an amplitude value, of the acceleration signal, at y2.

5. The method according to claim 3 or 4, wherein after the obtaining the maximum value point set and the minimum value point set, the method further comprises:
selecting a group of maximum value points from the maximum value point set; and
determining reasonableness of the group of maximum value points based on the preset reasonableness condition, wherein the preset reasonableness condition comprises at least one of the following items:
a first ratio of an average value of the combined acceleration to a quantity of turns is greater than a first threshold; or
a first standard deviation of a time interval between adjacent maximum value points is less than a second threshold; or
a second ratio of a quantity of valid maximum value points to a quantity of original maximum value points is greater than a third threshold; or
a second standard deviation of a difference between amplitude values of maximum value points is less than a fourth threshold; or
a quantity of turns corresponding to a speed less than a first value is not greater than a first quantity of turns, a quantity of turns corresponding to a speed greater than the first value and less than a second value is not greater than a second quantity of turns, and the first quantity of turns is less than the second quantity of turns.

6. The method according to claim 1, wherein the feature point set is an intersection point set, an intersection point in the intersection point set is an intersection point between the acceleration signal and an average value, and the average value is an average value of accelerations at all time points in the acceleration signal.

7. The method according to claim 1, wherein the acceleration signal is a gravity axis acceleration signal in the three-axis acceleration signal.

8. A wearable device to be worn on a user's leg and for calculating a tread frequency of a bicycle, wherein the wearable device comprises:
a three-axis accelerometer;
an obtaining module, configured to determine (201) a first state of an acceleration signal in a first time window, wherein the obtaining module is configured to:
obtain a three-axis acceleration signal;
calculate a combined acceleration of acceleration signals, of all axes, in the three-axis acceleration signal; and
determine the first state based on the combined acceleration; and
a processing module, configured to: when it is determined that the first state determined by the obtaining module is a non-riding state, record a quantity of turns in the first time window as 0, and, when it is preliminarily determined that the first state determined by the obtaining module comprises a riding state, determine (202) a feature point set from the acceleration signal, wherein time distribution of a feature point in the feature point set meets a preset periodic distribution condition, and amplitude meets a physical rule, and, when it is determined that a feature point subset in the feature point set meets a preset reasonableness condition, determine (203) that the first state is the riding state; and calculate (204), based on the feature point subset, a quantity of turns and a tread frequency that are in the first time window.
wherein after the determining (203) that the first state of the acceleration signal is the riding state, the processing module is further configured to:
determine a second state of the acceleration signal in a second time window and a third state of the acceleration signal in a third time window,
wherein the second time window, the third time window, and the first time window are adjacent and arranged in ascending order in time domain; and
when both the second state and the first state are riding states, and the third state is a non-riding state, correct the state of the acceleration signal in the third time window by:
calculating a second quantity of turns based on a feature point set of the acceleration signal in the third time window; and
when a ratio of the second quantity of turns to the first quantity of turns falls within a third value range, correcting the state of the acceleration signal in the third time window to the riding state, and updating a recorded quantity of turns in the third time window to the second quantity of turns.

9. The wearable device according to claim 8, wherein the feature point set comprises a maximum value point set and a minimum value point set, both time distribution of a maximum value point in the maximum value point set and time distribution of a minimum value point in the minimum value point set meet the preset periodic distribution condition, and the amplitude meets the physical rule.

10. The wearable device according to claim 9, wherein the processing module is configured to:
determine a first maximum value point set and a first minimum value point set from the acceleration signal;
calculate a time ratio and an amplitude ratio, of a first minimum value point and a second minimum value point, relative to a first maximum value point, wherein the first maximum value point is a maximum value point in the first maximum value point set, both the first minimum value point and the second minimum value point are minimum value points in the first minimum value point set, and the first minimum value point and the second minimum value point are extreme value points adjacent to the first maximum value point; and
when the time ratio falls within a first value range and the amplitude ratio falls within a second value range, determine that time distribution of the first maximum value point meets the preset reasonableness condition, and obtain the maximum value point set and the minimum value point set.

11. A computer-readable storage medium storing instructions which, when the program is executed by a computer, cause the computer to perform the method of claim 1.

12. A computer program product comprising instructions which, when the program is executed by a computer, cause the computer to perform the method of claim 1.

## Patentansprüche

1. Computerimplementiertes Verfahren zum Berechnen einer Trittfrequenz eines Fahrrads, wobei das Verfahren umfasst:
Bestimmen (201) eines ersten Zustands eines Beschleunigungssignals in einem ersten Zeitfenster, wobei der Schritt des Bestimmens (201) umfasst:
Erhalten eines dreiachsigen Beschleunigungssignals von einer tragbaren Vorrichtung, die an einem Bein eines Benutzers getragen wird;
Berechnen einer kombinierten Beschleunigung von Beschleunigungssignalen aller Achsen in dem dreiachsigen Beschleunigungssignal; und
Bestimmen des ersten Zustands basierend auf der kombinierten Beschleunigung;
wenn bestimmt wird, dass der erste Zustand ein Nicht-Fahrzustand ist, Aufzeichnen einer Anzahl von Umdrehungen in dem ersten Zeitfenster als 0;
wenn vorläufig bestimmt wird, dass der erste Zustand einen Fahrzustand umfasst, Bestimmen (202) einer Merkmalspunktmenge aus dem Beschleunigungssignal, wobei eine Zeitverteilung eines Merkmalspunkts in der Merkmalspunktmenge eine voreingestellte periodische Verteilungsbedingung erfüllt und eine Amplitude eine physikalische Regel erfüllt, und wenn bestimmt wird, dass eine Merkmalspunktteilmenge in der Merkmalspunktmenge eine voreingestellte Plausibilitätsbedingung erfüllt, Bestimmen (203), dass der erste Zustand der Fahrzustand ist; und
Berechnen (204), basierend auf der Merkmalspunktteilmenge, einer Anzahl von Umdrehungen und einer Trittfrequenz, die in dem ersten Zeitfenster liegen, wobei nach dem Bestimmen (203), dass der erste Zustand des Beschleunigungssignals der Fahrzustand ist, das Verfahren ferner umfasst:
Bestimmen eines zweiten Zustands des Beschleunigungssignals in einem zweiten Zeitfenster und eines dritten Zustands des Beschleunigungssignals in einem dritten Zeitfenster,
wobei das zweite Zeitfenster, das dritte Zeitfenster und das erste Zeitfenster angrenzend und in aufsteigender Reihenfolge in einer Zeitdomäne angeordnet sind; und
wenn sowohl der zweite Zustand als auch der erste Zustand Fahrzustände sind und der dritte Zustand ein Nicht-Fahrzustand ist, Korrigieren des Zustands des Beschleunigungssignals in dem dritten Zeitfenster durch:
Berechnen einer zweiten Anzahl von Umdrehungen basierend auf einer Merkmalspunktmenge des Beschleunigungssignals in dem dritten Zeitfenster; und
wenn ein Verhältnis der zweiten Anzahl von Umdrehungen zu der ersten Anzahl von Umdrehungen innerhalb eines dritten Wertbereichs fällt, Korrigieren des Zustands des Beschleunigungssignals in dem dritten Zeitfenster auf den Fahrzustand und Aktualisieren einer aufgezeichneten Anzahl von Umdrehungen in dem dritten Zeitfenster auf die zweite Anzahl von Umdrehungen.

2. Verfahren nach Anspruch 1, wobei die Merkmalspunktmenge eine Maximalwertpunktmenge und eine Minimalwertpunktmenge umfasst, sowohl die Zeitverteilung eines Maximalwertpunkts in der Maximalwertpunktmenge als auch die Zeitverteilung eines Minimalwertpunkts in der Minimalwertpunktmenge die voreingestellte periodische Verteilungsbedingung erfüllen und die Amplitude die physikalische Regel erfüllt.

3. Verfahren nach Anspruch 2, wobei das Bestimmen (202) einer Merkmalspunktmenge aus dem Beschleunigungssignal umfasst:
Bestimmen einer ersten Maximalwertpunktmenge und einer ersten Minimalwertpunktmenge aus dem Beschleunigungssignal;
Berechnen eines Zeitverhältnisses und eines Amplitudenverhältnisses eines ersten Minimalwertpunktes und eines zweiten Minimalwertpunktes relativ zu einem ersten Maximalwertpunkt, wobei der erste Maximalwertpunkt ein Maximalwertpunkt in der ersten Maximalwertpunktmenge ist, sowohl der erste Minimalwertpunkt als auch der zweite Minimalwertpunkt Minimalwertpunkte in der ersten Minimalwertpunktmenge sind und der erste Minimalwertpunkt und der zweite Minimalwertpunkt Extremwertpunkte, die an den ersten Maximalwertpunkt angrenzen, sind; und
wenn das Zeitverhältnis innerhalb eines ersten Wertbereichs fällt und das Amplitudenverhältnis innerhalb eines zweiten Wertbereichs fällt, Bestimmen, dass die Zeitverteilung des ersten Maximalwertpunkts die voreingestellte Plausibilitätsbedingung erfüllt, und Erhalten der Maximalwertpunktmenge und der Minimalwertpunktmenge.

4. Verfahren nach Anspruch 3, wobei das Zeitverhältnis [t(x) - t(y1)]/[t(y2) - t(x)] ist und das Amplitudenverhältnis [acc_f(x) - acc_f(y1)]/[acc_f(y2) - acc_f(x)] ist, wobei t(x) ein Zeitpunkt ist, der dem ersten Maximalwertpunkt x1 entspricht, t(y1) ein Zeitpunkt ist, der dem ersten Minimalwertpunkt y1 entspricht, t(y2) ein Zeitpunkt ist, der dem zweiten Minimalwertpunkt y2 entspricht, acc_f(x) einen Amplitudenwert des Beschleunigungssignals bei x darstellt, acc_f(y1) einen Amplitudenwert des Beschleunigungssignals bei y1 darstellt, und acc_f(y2) einen Amplitudenwert des Beschleunigungssignals bei y2 darstellt.

5. Verfahren nach Anspruch 3 oder 4, wobei nach dem Erhalten der Maximalwertpunktmenge und der Minimalwertpunktmenge das Verfahren ferner umfasst:
Auswählen einer Gruppe von Maximalwertpunkten aus der Maximalwertpunktmenge; und
Bestimmen der Plausibilität der Gruppe von Maximalwertpunkten basierend auf der voreingestellten Plausibilitätsbedingung, wobei die voreingestellte Plausibilitätsbedingung mindestens eines der folgenden Elemente umfasst:
ein erstes Verhältnis eines Durchschnittswerts der kombinierten Beschleunigung zu einer Anzahl von Umdrehungen ist größer als eine erste Schwelle; oder
eine erste Standardabweichung eines Zeitintervalls zwischen angrenzenden Maximalwertpunkten ist geringer als eine zweite Schwelle; oder
ein zweites Verhältnis einer Anzahl von gültigen Maximalwertpunkten zu einer Anzahl von ursprünglichen Maximalwertpunkten ist größer als eine dritte Schwelle; oder
eine zweite Standardabweichung einer Differenz zwischen Amplitudenwerten von Maximalwertpunkten ist geringer als eine vierte Schwelle; oder
eine Anzahl von Umdrehungen, die einer Geschwindigkeit entspricht, die geringer als ein erster Wert ist, ist nicht größer als eine erste Anzahl von Umdrehungen, eine Anzahl von Umdrehungen, die einer Geschwindigkeit entspricht, die größer als der erste Wert und geringer als ein zweiter Wert ist, ist nicht größer als eine zweite Anzahl von Umdrehungen, und die erste Anzahl von Umdrehungen ist geringer als die zweite Anzahl von Umdrehungen.

6. Verfahren nach Anspruch 1, wobei die Merkmalspunktmenge eine Schnittstellenpunktmenge ist, eine Schnittstelle in der Schnittstellenpunktmenge eine Schnittstelle zwischen dem Beschleunigungssignal und einem Durchschnittswert ist und der Durchschnittswert ein Durchschnittswert von Beschleunigungen zu allen Zeitpunkten in dem Beschleunigungssignal ist.

7. Verfahren nach Anspruch 1, wobei das Beschleunigungssignal ein Schwerkraftachsenbeschleunigungssignal in dem dreiachsigen Beschleunigungssignal ist.

8. Tragbare Vorrichtung, um an einem Bein eines Benutzers getragen zu werden und zum Berechnen einer Trittfrequenz eines Fahrrads, wobei die tragbare Vorrichtung umfasst:
einen dreiachsigen Beschleunigungsmesser;
ein Erhaltungsmodul, das konfiguriert ist, um einen ersten Zustand eines Beschleunigungssignals in einem ersten Zeitfenster zu bestimmen (201), wobei das Erhaltungsmodul konfiguriert ist zum:
Erhalten eines dreiachsigen Beschleunigungssignals;
Berechnen einer kombinierten Beschleunigung von Beschleunigungssignalen aller Achsen in dem dreiachsigen Beschleunigungssignal; und
Bestimmen des ersten Zustands basierend auf der kombinierten Beschleunigung; und
ein Verarbeitungsmodul, das konfiguriert ist zum: wenn bestimmt wird, dass der erste Zustand, der durch das Erhaltungsmodul bestimmt wird, ein Nicht-Fahrzustand ist, Aufzeichnen einer Anzahl von Umdrehungen in dem ersten Zeitfenster als 0, und, wenn vorläufig bestimmt wird, dass der erste Zustand, der durch das Erhaltungsmodul bestimmt wird, einen Fahrzustand umfasst, Bestimmen (202) einer Merkmalspunktmenge aus dem Beschleunigungssignal, wobei eine Zeitverteilung eines Merkmalspunkts in der Merkmalspunktmenge eine voreingestellte periodische Verteilungsbedingung erfüllt und eine Amplitude eine physikalische Regel erfüllt, und, wenn bestimmt wird, dass eine Merkmalspunktteilmenge in der Merkmalspunktmenge eine voreingestellte Plausibilitätsbedingung erfüllt, Bestimmen (203), dass der erste Zustand der Fahrzustand ist; und Berechnen (204), basierend auf der Merkmalspunktteilmenge, einer Anzahl von Umdrehungen und einer Trittfrequenz, die in dem ersten Zeitfenster liegen.
wobei nach dem Bestimmen (203), dass der erste Zustand des Beschleunigungssignals der Fahrzustand ist, das Verarbeitungsmodul ferner konfiguriert ist zum:
Bestimmen eines zweiten Zustands des Beschleunigungssignals in einem zweiten Zeitfenster und eines dritten Zustands des Beschleunigungssignals in einem dritten Zeitfenster,
wobei das zweite Zeitfenster, das dritte Zeitfenster und das erste Zeitfenster angrenzend und in aufsteigender Reihenfolge in einer Zeitdomäne angeordnet sind; und
wenn sowohl der zweite Zustand als auch der erste Zustand Fahrzustände sind und der dritte Zustand ein Nicht-Fahrzustand ist, Korrigieren des Zustands des Beschleunigungssignals in dem dritten Zeitfenster durch:
Berechnen einer zweiten Anzahl von Umdrehungen basierend auf einer Merkmalspunktmenge des Beschleunigungssignals in dem dritten Zeitfenster; und
wenn ein Verhältnis der zweiten Anzahl von Umdrehungen zu der ersten Anzahl von Umdrehungen innerhalb eines dritten Wertbereichs fällt, Korrigieren des Zustands des Beschleunigungssignals in dem dritten Zeitfenster auf den Fahrzustand und Aktualisieren einer aufgezeichneten Anzahl von Umdrehungen in dem dritten Zeitfenster auf die zweite Anzahl von Umdrehungen.

9. Tragbare Vorrichtung nach Anspruch 8, wobei die Merkmalspunktmenge eine Maximalwertpunktmenge und eine Minimalwertpunktmenge umfasst, wobei sowohl die Zeitverteilung eines Maximalwertpunkts in der Maximalwertpunktmenge als auch die Zeitverteilung eines Minimalwertpunkts in der Minimalwertpunktmenge die voreingestellte periodische Verteilungsbedingung erfüllen und die Amplitude die physikalische Regel erfüllt.

10. Tragbare Vorrichtung nach Anspruch 9, wobei das Verarbeitungsmodul konfiguriert ist zum:
Bestimmen einer ersten Maximalwertpunktmenge und einer ersten Minimalwertpunktmenge aus dem Beschleunigungssignal;
Berechnen eines Zeitverhältnisses und eines Amplitudenverhältnisses eines ersten Minimalwertpunktes und eines zweiten Minimalwertpunktes relativ zu einem ersten Maximalwertpunkt, wobei der erste Maximalwertpunkt ein Maximalwertpunkt in der ersten Maximalwertpunktmenge ist, sowohl der erste Minimalwertpunkt als auch der zweite Minimalwertpunkt Minimalwertpunkte in der ersten Minimalwertpunktmenge sind und der erste Minimalwertpunkt und der zweite Minimalwertpunkt Extremwertpunkte, die an den ersten Maximalwertpunkt angrenzen, sind; und
wenn das Zeitverhältnis innerhalb eines ersten Wertbereichs fällt und das Amplitudenverhältnis innerhalb eines zweiten Wertbereichs fällt, Bestimmen, dass die Zeitverteilung des ersten Maximalwertpunkts die voreingestellte Plausibilitätsbedingung erfüllt, und Erhalten der Maximalwertpunktmenge und der Minimalwertpunktmenge.

11. Computerlesbares Speichermedium, das Anweisungen speichert, die, wenn das Programm durch einen Computer ausgeführt wird, den Computer veranlassen, das Verfahren nach Anspruch 1 durchzuführen.

12. Computerprogramm, das Anweisungen umfasst, die, wenn das Programm durch einen Computer ausgeführt wird, den Computer veranlassen, das Verfahren nach Anspruch 1 durchzuführen.

## Revendications

1. Procédé mis en œuvre par ordinateur pour le calcul d'une fréquence de pédalage d'un vélo, dans lequel le procédé comprend :
la détermination (201) d'un premier état d'un signal d'accélération dans une première fenêtre temporelle, dans lequel l'étape de détermination (201) comprend :
l'obtention d'un signal d'accélération à trois axes à partir d'un dispositif portable porté sur une jambe d'un utilisateur ;
le calcul d'une accélération combinée de signaux d'accélération, de tous les axes, dans le signal d'accélération à trois axes ; et
la détermination du premier état sur la base de l'accélération combinée ;
lorsqu'il est déterminé que le premier état est un état de non-conduite, l'enregistrement d'un nombre de tours dans la première fenêtre temporelle comme étant de 0 ;
lorsqu'il est déterminé au préalable que le premier état comprend un état de conduite, la détermination (202) d'un ensemble de points caractéristiques à partir du signal d'accélération, dans lequel une distribution temporelle d'un point caractéristique dans l'ensemble de points caractéristiques satisfait à une condition de distribution périodique prédéfinie, et qu'une amplitude satisfait à une règle physique, et lorsqu'il est déterminé qu'un sous-ensemble de points caractéristiques dans l'ensemble de points caractéristiques satisfait à une condition de caractère raisonnable prédéfinie, la détermination (203) du fait que le premier état est l'état de conduite ; et
le calcul (204), sur la base du sous-ensemble de points caractéristiques, d'un nombre de tours et d'une fréquence de pédalage qui sont dans la première fenêtre temporelle, dans lequel, après la détermination (203) du fait que le premier état du signal d'accélération est l'état de conduite, le procédé comprend en outre :
la détermination d'un deuxième état du signal d'accélération dans une deuxième fenêtre temporelle et d'un troisième état du signal d'accélération dans une troisième fenêtre temporelle,
dans lequel la deuxième fenêtre temporelle, la troisième fenêtre temporelle et la première fenêtre temporelle sont adjacentes et disposées dans un ordre croissant dans le domaine temporel ; et
lorsqu'à la fois le deuxième état et le premier état sont des états de conduite, et que le troisième état est un état de non-conduite, la correction de l'état du signal d'accélération dans la troisième fenêtre temporelle par :
le calcul d'un second nombre de tours sur la base d'un ensemble de points caractéristiques du signal d'accélération dans la troisième fenêtre temporelle ; et
lorsqu'un rapport du second nombre de tours au premier nombre de tours se situe dans une troisième plage de valeurs, la correction de l'état du signal d'accélération dans la troisième fenêtre temporelle pour correspondre à l'état de conduite, et la mise à jour d'un nombre enregistré de tours dans la troisième fenêtre temporelle pour correspondre au second nombre de tours.

2. Procédé selon la revendication 1, dans lequel l'ensemble de points caractéristiques comprend un ensemble de points de valeur maximale et un ensemble de points de valeur minimale, à la fois une distribution temporelle d'un point de valeur maximale dans l'ensemble de points de valeur maximale et une distribution temporelle d'un point de valeur minimale dans l'ensemble de points de valeur minimale satisfont à la condition de distribution périodique prédéfinie, et l'amplitude satisfait à la règle physique.

3. Procédé selon la revendication 2, dans lequel la détermination (202) d'un ensemble de points caractéristiques à partir du signal d'accélération comprend :
la détermination d'un premier ensemble de points de valeur maximale et d'un premier ensemble de points de valeur minimale à partir du signal d'accélération ;
le calcul d'un rapport temporel et d'un rapport d'amplitude, d'un premier point de valeur minimale et d'un second point de valeur minimale, par rapport à un premier point de valeur maximale, dans lequel le premier point de valeur maximale est un point de valeur maximale dans le premier ensemble de points de valeur maximale, à la fois le premier point de valeur minimale et le second point de valeur minimale sont des points de valeur minimale dans le premier ensemble de points de valeur minimale, et le premier point de valeur minimale et le second point de valeur minimale sont des points de valeur extrême adjacents au premier point de valeur maximale ; et
lorsque le rapport temporel se situe dans une première plage de valeurs et que le rapport d'amplitude se situe dans une deuxième plage de valeurs, la détermination du fait qu'une distribution temporelle du premier point de valeur maximale satisfait à la condition de caractère raisonnable prédéfinie, et l'obtention de l'ensemble de points de valeur maximale et de l'ensemble de points de valeur minimale.

4. Procédé selon la revendication 3, dans lequel le rapport temporel est
[t(x) - t(y1)]/[t(y2) - t(x)], et le rapport d'amplitude est
[acc_f(x) - acc_f(y1)]/[acc_f(y2) - acc_f(x)], dans lequel
t(x) représente un point temporel correspondant au premier point de valeur maximale x1, t(y1) représente un point temporel correspondant au premier point de valeur minimale y1, t(y2) représente un point temporel correspondant au second point de valeur minimale y2, acc_f(x) représente une valeur d'amplitude, du signal d'accélération, à x, acc_f(y1) représente une valeur d'amplitude, du signal d'accélération, à y1, et acc_f(y2) représente une valeur d'amplitude, du signal d'accélération, à y2.

5. Procédé selon la revendication 3 ou 4, dans lequel, après l'obtention de l'ensemble de points de valeur maximale et de l'ensemble de points de valeur minimale, le procédé comprend en outre :
la sélection d'un groupe de points de valeur maximale à partir de l'ensemble de points de valeur maximale ; et
la détermination d'un caractère raisonnable du groupe de points de valeur maximale sur la base de la condition de caractère raisonnable prédéfinie, dans lequel la condition de caractère raisonnable prédéfinie comprend au moins l'un des éléments suivants :
un premier rapport d'une valeur moyenne de l'accélération combinée à un nombre de tours est supérieur à un premier seuil ; ou
un premier écart-type d'un intervalle de temps entre des points de valeur maximale adjacents est inférieur à un deuxième seuil ; ou
un second rapport d'un nombre de points de valeur maximale valides à un nombre de points de valeur maximale d'origine est supérieur à un troisième seuil ; ou
un second écart-type d'une différence entre des valeurs d'amplitude de points de valeur maximale est inférieur à un quatrième seuil ; ou
un nombre de tours correspondant à une vitesse inférieure à une première valeur n'est pas supérieur à un premier nombre de tours, un nombre de tours correspondant à une vitesse supérieure à la première valeur et inférieure à une seconde valeur n'est pas supérieur à un second nombre de tours, et le premier nombre de tours est inférieur au second nombre de tours.

6. Procédé selon la revendication 1, dans lequel l'ensemble de points caractéristiques est un ensemble de points d'intersection, un point d'intersection dans l'ensemble de points d'intersection est un point d'intersection entre le signal d'accélération et une valeur moyenne, et la valeur moyenne est une valeur moyenne d'accélérations à tous les points temporels dans le signal d'accélération.

7. Procédé selon la revendication 1, dans lequel le signal d'accélération est un signal d'accélération d'axe de gravité dans le signal d'accélération à trois axes.

8. Dispositif portable à porter sur une jambe d'un utilisateur et permettant de calculer une fréquence de pédalage d'un vélo, dans lequel le dispositif portable comprend :
un accéléromètre à trois axes ;
un module d'obtention, configuré pour déterminer (201) un premier état d'un signal d'accélération dans une première fenêtre temporelle, dans lequel le module d'obtention est configuré pour :
obtenir un signal d'accélération à trois axes ;
calculer une accélération combinée de signaux d'accélération, de tous les axes, dans le signal d'accélération à trois axes ; et
déterminer le premier état sur la base de l'accélération combinée ; et
un module de traitement, configuré pour : lorsqu'il est déterminé que le premier état déterminé par le module d'obtention est un état de non-conduite, enregistrer un nombre de tours dans la première fenêtre temporelle comme étant de 0, et, lorsqu'il est déterminé au préalable que le premier état déterminé par le module d'obtention comprend un état de conduite, déterminer (202) un ensemble de points caractéristiques à partir du signal d'accélération, dans lequel une distribution temporelle d'un point caractéristique dans l'ensemble de points caractéristiques satisfait à une condition de distribution périodique prédéfinie, et une amplitude satisfait à une règle physique, et, lorsqu'il est déterminé qu'un sous-ensemble de points caractéristiques dans l'ensemble de points caractéristiques satisfait à une condition de caractère raisonnable prédéfinie, déterminer (203) que le premier état est l'état de conduite ; et calculer (204), sur la base du sous-ensemble de points caractéristiques, un nombre de tours et une fréquence de pédalage qui sont dans la première fenêtre temporelle.
dans lequel, après la détermination (203) du fait que le premier état du signal d'accélération est l'état de conduite, le module de traitement est en outre configuré pour :
déterminer un deuxième état du signal d'accélération dans une deuxième fenêtre temporelle et un troisième état du signal d'accélération dans une troisième fenêtre temporelle,
dans lequel la deuxième fenêtre temporelle, la troisième fenêtre temporelle et la première fenêtre temporelle sont adjacentes et disposées dans un ordre croissant dans le domaine temporel ; et
lorsqu'à la fois le deuxième état et le premier état sont des états de conduite, et que le troisième état est un état de non-conduite, corriger l'état du signal d'accélération dans la troisième fenêtre temporelle par :
le calcul d'un second nombre de tours sur la base d'un ensemble de points caractéristiques du signal d'accélération dans la troisième fenêtre temporelle ; et
lorsqu'un rapport du second nombre de tours au premier nombre de tours se situe dans une troisième plage de valeurs, la correction de l'état du signal d'accélération dans la troisième fenêtre temporelle pour correspondre à l'état de conduite, et la mise à jour d'un nombre enregistré de tours dans la troisième fenêtre temporelle pour correspondre au second nombre de tours.

9. Dispositif portable selon la revendication 8, dans lequel l'ensemble de points caractéristiques comprend un ensemble de points de valeur maximale et un ensemble de points de valeur minimale, à la fois une distribution temporelle d'un point de valeur maximale dans l'ensemble de points de valeur maximale et une distribution temporelle d'un point de valeur minimale dans l'ensemble de points de valeur minimale satisfont à la condition de distribution périodique prédéfinie, et l'amplitude satisfait à la règle physique.

10. Dispositif portable selon la revendication 9, dans lequel le module de traitement est configuré pour :
déterminer un premier ensemble de points de valeur maximale et un premier ensemble de points de valeur minimale à partir du signal d'accélération ;
calculer un rapport temporel et un rapport d'amplitude, d'un premier point de valeur minimale et d'un second point de valeur minimale, par rapport à un premier point de valeur maximale, dans lequel le premier point de valeur maximale est un point de valeur maximale dans le premier ensemble de points de valeur maximale, à la fois le premier point de valeur minimale et le second point de valeur minimale sont des points de valeur minimale dans le premier ensemble de points de valeur minimale, et le premier point de valeur minimale et le second point de valeur minimale sont des points de valeur extrême adjacents au premier point de valeur maximale ; et
lorsque le rapport temporel se situe dans une première plage de valeurs et que le rapport d'amplitude se situe dans une deuxième plage de valeurs, déterminer qu'une distribution temporelle du premier point de valeur maximale satisfait à la condition de caractère raisonnable prédéfinie, et obtenir l'ensemble de points de valeur maximale et l'ensemble de points de valeur minimale.

11. Support de stockage lisible par ordinateur stockant des instructions qui, lorsque le programme est exécuté par un ordinateur, amènent l'ordinateur à réaliser le procédé selon la revendication 1.

12. Produit-programme d'ordinateur comprenant des instructions qui, lorsque le programme est exécuté par un ordinateur, amènent l'ordinateur à réaliser le procédé selon la revendication 1.
